# EUROPEAN PATENT APPLICATION

(11) **EP 4 613 261 A1**
(43) Date of publication of application: **10.09.2025**
(21) Application number: 23886249.4
(22) Date of filing: 01.11.2023
(51) Int. Cl.: A61K 8/73, A61K 8/44, A61Q 19/00

(54) **COMPLEX FOR IMPROVING SKIN CONDITION**

(30) Priority: 02.11.2022 KR 20220144642; 31.10.2023 KR 20230148281
(71) Applicant: LG H&H Co., Ltd., Seoul 03184 (KR)
(72) Inventor: JEON, Hyung Joon, Seoul 07795 (KR); KWON, Koo Chul, Seoul 07795 (KR); SHIN, Yong Won, Seoul 07795 (KR); WON, Jong Gu, Seoul 07795 (KR); PARK, Sang Wook, Seoul 07795 (KR); OH, Yoon Seo, Seoul 07795 (KR); KIM, Mi Sun, Seoul 07795 (KR)
(74) Representative: Goddar, Heinz J.
(86) International application number: PCT/KR2023/017215
(87) International publication number: WO 2024/096553

(57) **Abstract**

The present invention relates to a composition comprising a glycosaminoglycans (GAGs)-amino acid complex (GAGs-AA complex) as an active ingredient. In addition, the cosmetic composition according to the present invention made to include a GAGs-AA complex by means of physicochemical interaction as an active ingredient, and thus can exhibit remarkable effects for brightening skin, ameliorating wrinkles, improving elasticity, moisturizing, improving skin barriers, enhancing beneficial bacteria of the skin, or soothing the skin.

## Description

### [Technical Field]

The present invention relates to a glycosaminoglycan (GAG) component, in particular, a polyion complex between chondroitin sulfate (CS) and an amino acid (AA) (a GAGs-AA complex or CS-AA complex).

### [Background Art]

In the field of beauty, the areas of greatest concern are wrinkles, skin pigmentation, loss of skin elasticity, skin drying, hair loss and lack of shine in hair associated with aging.

In particular, the skin undergoes various changes through aging. Before aging begins in the skin, skin cells are highly physiologically active, and therefore fibroblasts in the dermis are active in the synthesis and metabolism of collagen, which is important for skin elasticity and suppression of wrinkle formation. Further, glycosaminoglycans (GAGs), glycoproteins that contain hyaluronic acid, which is essential for maintaining skin moisture, are also actively synthesized, imparting elasticity, softness, flexibility, and moisturizing properties to the skin. In addition, in the epidermis, the proliferation of basal layer cells is active, and adhesion proteins such as laminin, integrin, and desmosome, which strengthen the bonds between skin cells, are produced in a balanced way to strengthen skin cell tissue, thereby keeping the skin healthy. However, the skin of modern people is exposed to various harmful external environmental factors and stress, resulting in various types of damage, and as the skin ages, the physiological activity of the skin decreases, the recovery rate of damaged skin is slowed down, and various skin aging phenomena such as loss of elasticity, dryness, wrinkle formation, pigmentation, senile plaque, and dullness appear. Therefore, various cosmetic products have been developed to ameliorate such aging symptoms.

Chondroitin sulfate is one of the various components that make up the human body, particularly the joints, and has been studied as a biotransplant material, and the like, and is also used as an ingestible therapeutic agent for arthritis. However, chondroitin sulfate has low skin absorption or skin permeability due to a large molecular weight, and for this reason, when it is included in a skin external preparation, the skin improvement effect to be felt is not significant, and therefore its use in the cosmetic field is limited. In this regard, biopharmaceuticals, drug delivery system and tissue engineering scaffolds that have temperature- and pH-sensitive sol-gel transition properties in aqueous media, including chitosan-polymer complexes, are known (Korean Patent No. 10-1335624). However, this approach has a problem in that the inherent effects of each substance that constitutes the complex are lost during the complex formation process.

Furthermore, a cosmetic composition including chondroitin sulfate to exhibit the effects of promoting skin keratinocyte differentiation, strengthening the skin barrier function, and moisturizing the skin is known (Korean Patent No. 10-1445644). However, when chondroitin sulfate is used alone or as a simple mixture as an active ingredient as described above, there is a problem in that the limitations in the related art are not resolved.

Therefore, the present invention has been made in an effort to form a complex in which chondroitin sulfate is bound to an amino acid monomer having a cationic functional group through electrostatic interaction, thereby increasing the skin absorption of chondroitin sulfate and maximizing the skin improving efficacy of chondroitin sulfate.
In this regard, a chondroitin sulfate-amino acid complex (CS-AA complex) was
prepared, and it was confirmed, based on the zeta potential value measured by DLS, that chondroitin sulfate, which previously aggregated linearly to form a wall-like structure, adopted a particulate form, thereby completing the present invention.

### [Related Art Documents]

### [Patent Documents]

Korean Patent No. 10-0365242
Korean Patent No. 10-1445644

### [Disclosure]

### [Technical Problem]

Under the background described above, the present inventors have confirmed that a glycosaminoglycans (GAGs)-amino acid complex exhibits remarkable effects for brightening skin, ameliorating wrinkles, improving elasticity, moisturizing, improving skin barriers, enhancing beneficial bacteria of the skin, or soothing the skin by means of physicochemical interaction, thereby completing the present invention.

An object of the present invention is to provide a cosmetic composition comprising a glycosaminoglycan (GAG)-amino acid complex as an active ingredient.

### [Technical Solution]

As a means for solving the above problem, the present invention provides a glycosaminoglycans (GAGs)-amino acid complex as a complex for skin improvement.

One aspect of the present invention provides an excellent skin improvement method, including brightening skin, ameliorating wrinkles, improving elasticity, moisturizing, improving skin barriers, enhancing beneficial bacteria of the skin, or soothing the skin, the method including treating the skin with a GAGs-amino acid complex.

Another aspect of the present invention provides an excellent skin improvement method, including brightening skin, ameliorating wrinkles, improving elasticity, moisturizing, improving skin barriers, enhancing beneficial bacteria of the skin, or soothing the skin, the method including administering a GAGs-amino acid complex to a subject.

Still another aspect of the present invention provides a use of a GAGs-amino acid complex for the preparation of a cosmetic composition for an excellent skin improvement, such as brightening skin, ameliorating wrinkles, improving elasticity, moisturizing, improving skin barriers, enhancing beneficial bacteria of the skin, or soothing the skin.

**In** the present invention, "skin improvement" may refer to brightening skin; moisturizing skin; improving skin elasticity; ameliorating skin wrinkles; improving skin barriers; enhancing beneficial bacteria of the skin or soothing the skin; and the like, but is not limited thereto.

**In** the present invention, "brightening skin" refers not only to lightening the skin tone, but also to alleviating or ameliorating melasma, freckles, and hyperpigmentation caused by ultraviolet rays, hormones, or genetics.

**In** the present invention, "moisturizing skin" refers to supplying moisture to the skin that has become dry, rough, and keratinized due to dry air and irritation, blocking the evaporation of moisture to maintain the flexibility of the skin, and maintaining a smooth surface.

The moisturizing of the skin may relate to one or more selected from the group consisting of, for example, atopic dermatitis, psoriasis, xeroderma, eczema, and xeroderma pigmentosum, but is not limited thereto.

In the present invention, "ameliorating skin wrinkles" refers to preventing, suppressing or inhibiting the generation of wrinkles on the skin, or alleviating already generated wrinkles.

In the present invention, "improving skin barriers" refers to strengthening the barriers of the stratum corneum located in the outermost layer of the skin, and refers to improving the luster of the skin, improving keratin condition, protecting the skin from external factors, and improving the ability to maintain skin homeostasis.

In the present invention, "beneficial bacteria of the skin" refers to a strain of skin resident flora that is useful for alleviating inflammation, regulating inflammation associated with psoriasis, suppressing the growth of harmful bacteria, and inhibiting the growth of tumors. The beneficial bacteria of the skin may be, for example, *Staphylococcus epidermidis,* and the like, but are not limited thereto. "Harmful bacteria of the skin" refers to bacteria that cause inflammatory responses or acne on the skin, and the like. The harmful bacteria of the skin may be, for example, *Propionibacterium acnes, Corynebacterium minutissimum, Staphylococcus aureus,* and the like, but are not limited thereto.

In the present invention, "enhancing beneficial bacteria of the skin" refers to promoting or improving the growth of the aforementioned beneficial bacteria of the skin, and improving the growth of beneficial bacteria also includes preventing the growth of beneficial bacteria from being inhibited. For the purposes of the present invention, the enhancing of the beneficial bacteria of the skin may be the enhancement of *Staphylococcus epidermidis,* but is not limited thereto.

In the present invention, "soothing the skin" refers to suppressing or inhibiting skin inflammation, erythema, or skin tingling caused by external stimuli, or soothing existing skin irritation symptoms.

Hereinafter, the configuration of the present invention will be described in detail.

In the present invention, for the glycosaminoglycans (GAGs), it is possible to use at least one selected from the group consisting of chondroitin sulfate, carrageenan, dextran sulfate, dermatan sulfate, heparan sulfate, keratan sulfate, heparin sulfate, and hyaluronic acid. In the present invention, among GAGs, it is possible to use at least one selected from the group consisting of chondroitin sulfate, dextran sulfate, dermatan sulfate, heparan sulfate, keratan sulfate, and heparin sulfate, which are sulfated GAGs (sGAGs), and preferably, chondroitin sulfate and/or dextran sulfate may be used. Dextran sulfate is an isomer of chondroitin sulfate.

In the present invention, for the amino acid, it is possible to use at least one selected from the group consisting of serine, proline, threonine, cysteine, glycine, alanine, valine, leucine, isoleucine, aspartic acid, glutamic acid, glutamine, arginine, lysine, tyrosine, methionine, phenylalanine, histidine, tryptophan, and carnitine. As a preferred amino acid, it is possible to use at least one of the group consisting of glycine, serine, threonine, alanine, valine, leucine, cysteine, glutamine, arginine, and carnitine, which have a -NH₃⁺ functional group, that is, an -amino group, which is protonated under biological conditions to exhibit amphoteric properties. More preferably, it is possible to use at least one selected from the group consisting of arginine, glutamine, lysine, and carnitine, which are amino acids having a benzene ring structure or a small number of branched chains, thus having a structurally small size and is likely to interact with GAGs. Most preferably, arginine and glutamine may be used.

Commonly used GAGs have a large molecular weight, and therefore have a low bioabsorption rate and are difficult to apply at high concentrations to cosmetics, and have limitations in the formulations to which they can be applied.

In the present invention, GAGs are not used alone, but are prepared and used as a complex that undergoes physicochemical interaction with amino acids. The cosmetic composition according to the present invention may improve the bioabsorption rate of GAGs by including a GAGs-amino acid complex, and may exhibit an effect that is more improved than the inherent efficacy of GAGs. Specifically, the GAGs-amino acid complex according to the present invention may improve the skin improvement effect 1.2-fold or more, 1.5-fold or more, 2-fold or more, 5-fold or more, 10-fold or more, or 15-fold or more compared to when GAGs are used in an amount that is the same as the amount of GAGs in the complex. Further, the complex according to the present invention may improve the skin improvement effect 1.2-fold or more, 1.3-fold or more, 1.5-fold or more, 1.6-fold or more, 2-fold or more, 3-fold or more, or 5-fold or more compared to a simple mixture of GAGs and amino acids in the same amounts as those in the complex.

In addition, the cosmetic composition according to the present invention may improve the stability of the composition by including a GAGs-amino acid complex.

Furthermore, in a specific embodiment of the present invention, as a result of preparing a GAGs-amino acid complex to evaluate its skin improvement effects *in vitro* and *in vivo,* it was confirmed that the complex can be used as a material for improving a skin condition by confirming that the complex has excellent effects of increasing the synthesis of collagen, increasing cell activity, increasing the synthesis of hyaluronic acid, suppressing the production of inflammatory mediators, moisturizing, brightening, improving skin texture, improving pores, and enhancing beneficial bacteria of the skin among the skin resident flora.

The GAGs-amino acid complex is a complex formed by two components by means of physicochemical interaction, and is a polyion complex. **In** other words, the physicochemical interaction of the GAGs-amino acid complex refers to an intermolecular interaction that is carried out in a state in which the individual unique molecular structures are maintained based on hydrophobic interaction, interaction between charges, or hydrogen bond.
A ratio between materials required for the GAGs-amino acid complex may be
determined depending on the charge of each molecule of the GAGs-amino acid complex, the type and number of functional groups of the molecule, and the polarity of the molecule or functional group. Further, the ratio between materials required for the GAGs-amino acid complex may be determined according to the size or similarity of the molecules.

In the present invention, the GAGs-amino acid complex may be mixed at a molar ratio of GAGs:amino acid=1:0.001 to 10, for example, 1:0.001 to 10, 1:0.01 to 1:8, 1:0.1 to 1:5, or 1:1 to 3. It was confirmed in Experimental Example 1 that the GAGs-amino acid complex was stable within the corresponding molar ratio range.

When the molar ratio is outside of the above molar ratio range, the remaining molecules that do not form a complex may exceed their solubility in water and may be precipitated at low temperatures.

In addition, the present invention relates to a cosmetic composition including a GAGs-amino acid complex as an active ingredient.

In the present invention, the cosmetic composition may be in the form of a general emulsion formulation and a general solubilized formulation. For example, the cosmetic composition may have a formulation such as a lotion such as an emollient lotion or nourishing lotion, an emulsion such as a facial lotion or a body lotion, a cream such as a nourishing cream, a moisture cream or an eye cream, an essence, a cosmetic ointment, a balm, a spray, a gel, a pack, sunscreen, a makeup base, a foundation such as a liquid, solid or spray type, a powder, a makeup remover such as a cleansing cream, a cleansing lotion, or a cleansing oil, and a cleansing agent such as a cleansing foam, a soap, and a body wash.

Furthermore, the cosmetics may additionally contain auxiliary agents typically used in cosmetology, such as a fatty substance, an organic solvent, a solubilizing agent, a thickener, a gelling agent, a softener, an antioxidant, a suspending agent, a stabilizer, a foaming agent, an odorant, a surfactant, water, an ionic or a nonionic emulsifier, a filler, a metal ion sequestering agent, a chelating agent, a preservative, a vitamin, a blocking agent, a wetting agent, an essential oil, a dye, a pigment, a hydrophilic or lipophilic active agent, a lipid vesicle, or any other ingredient typically used in cosmetics.

The cosmetic formulation may include a relatively high concentration of the composition of the present invention in the case of wash-off type cosmetics such as makeup removers and cleansing agents, in which the active ingredient remains on the skin for a short period of time. **In** contrast, in the case of leave-on type cosmetics such as lotions, emulsions, creams, and essences, in which the active ingredient remains on the skin for a long period of time, it may be acceptable to include a lower concentration of the composition of the present invention than in wash-off type cosmetics. Without being limited thereto, in a specific embodiment of the present invention, the composition of the present invention may include the GAGs-amino acid complex in an amount of 0.0001 to 30 parts by weight, for example, 0.0001 to 0.001 parts by weight, 0.001 to 0.01 parts by weight, 0.01 to 0.05 parts by weight, 0.05 to 0.1 parts by weight, 0.1 to 0.5 parts by weight, 0.5 to 1 part by weight, 1 to 1.5 parts by weight, 1.5 to 3 parts by weight, 3 to 5 parts by weight, 5 to 8 parts by weight, 8 to 10 parts by weight, 10 to 15 parts by weight, 15 to 20 parts by weight, 20 to 25 parts by weight, or 25 to 30 parts by weight, based on 100 parts by weight of the entire composition.

When the GAGs-amino acid complex according to the present invention is included in an amount less than 0.0001 parts by weight based on 100 parts by weight of the entire composition, sufficient effects for bright ening skin, ameliorating wrinkles, improving elasticity, moisturizing, improving skin barriers or soothing the skin cannot be expected, and when it is included in an amount exceeding 30 parts by weight, undesired reactions such as allergies may occur or there may be a problem with skin safety, and the range is intended to prevent this.

Each of the above components included in the cosmetic composition according to the present invention may be included in the cosmetic composition according to the present invention within the range not exceeding the maximum usage amount stipulated in the cosmetic safety standard of each country.

Further, the present invention provides a method for preparing a cosmetic composition including a GAGs-amino acid complex.

Hereinafter, the preparation method of the present invention will be described in detail.

The preparation method of the present invention may include: mixing an amino acid and a solvent to prepare a first mixture; performing first heating and stirring on the first mixture; adding GAGs and performing second heating and stirring when the first heating and stirring is completed; homogenizing the mixture to prepare a GAGs-amino acid complex when the second heating and stirring is completed; and adding the GAGs-amino acid complex to prepare a cosmetic composition.

In the preparing of the first mixture, the amino acid may include at least one selected from the group consisting of serine, proline, threonine, cysteine, glycine, alanine, valine, leucine, isoleucine, aspartic acid, glutamic acid, glutamine, arginine, lysine, tyrosine, methionine, phenylalanine, histidine, tryptophan, and carnitine. As a preferred amino acid, it is possible to use at least one of the group consisting of glycine, serine, threonine, alanine, valine, leucine, cysteine, glutamine, arginine, and carnitine, which have a -NH₃⁺ functional group, that is, an -amino group, which is protonated under biological conditions to exhibit amphoteric properties. More preferably, it is possible to use at least one selected from the group consisting of arginine, glutamine, lysine, and carnitine, which are amino acids having a benzene ring structure or a small number of branched chains, thus having a structurally small size and is likely to interact with GAGs. Most preferably, arginine and glutamine may be used.

The performing of the first heating and stirring includes stirring at 500 to 1500 rpm at 30 to 80°C for 3 to 30 minutes.

When the temperature of the performing of the first heating and stirring is less than 30°C, the complex is not sufficiently formed, and when the temperature exceeds 70°C, the complex is decomposed, resulting in a decrease in the skin improvement effect.

In the preparing of the GAGs-amino acid complex, GAGs may include at least one selected from the group consisting of chondroitin sulfate, carrageenan, dextran sulfate, dermatan sulfate, heparan sulfate, keratan sulfate, heparin sulfate, and hyaluronic acid.

In the preparing of the GAGs-amino acid complex, the GAGs may be mixed at a molar ratio of GAGs:amino acid=1:0.001 to 10, for example, 1:0.001 to 10, 1:0.01 to 1:8, 1:0.1 to 1:5, or 1:1 to 1:3.

The preparing of the GAGs-amino acid complex includes stirring at 500 to 1500 rpm at 30 to 80°C for 5 to 30 minutes.

The preparing of the GAGs-amino acid complex may further include removing the solvent in a subsequent process. The removing of the solvent may use a method for drying the cosmetic composition, for example, a freeze-drying method. Through the removing of the solvent, the GAGs-amino acid complex may be obtained as a solid.

In the preparing of the cosmetic composition, the GAGs-amino acid complex may be added in an amount of 0.0001 to 30 parts by weight, for example, 0.0001 to 0.001 parts by weight, 0.001 to 0.01 parts by weight, 0.01 to 0.05 parts by weight, 0.05 to 0.1 parts by weight, 0.1 to 0.5 parts by weight, 0.5 to 1 part by weight, 1 to 1.5 parts by weight, 1.5 to 3 parts by weight, 3 to 5 parts by weight, 5 to 8 parts by weight, 8 to 10 parts by weight, 10 to 15 parts by weight, 15 to 20 parts by weight, 20 to 25 parts by weight, or 25 to 30 parts by weight, based on 100 parts by weight of the entire composition.

When the preparing of the GAGs-amino acid complex is subjected to the removing of the solvent in a subsequent process, the preparing of the cosmetic composition may include the GAGs-amino acid complex in an amount of 50 to 100 parts by weight, for example, 90 to 100 parts by weight, 95 to 100 parts by weight, or 99 to 100 parts by weight, based on 100 parts by weight of the entire composition.

### [Advantageous Effects]

The cosmetic composition according to the present invention made to include a glycosaminoglycans (GAGs)-amino acid complex by means of physicochemical interaction as an active ingredient, and thus can exhibit remarkable effects for bright ening skin, ameliorating wrinkles, improving elasticity, moisturizing, improving skin barriers, enhancing beneficial bacteria of the skin, or soothing the skin.

### [Description of Drawings]

FIG. 1 shows TEM analysis results of a) chondroitin sulfate alone, b) a simple mixture of chondroitin sulfate and glutamine, c) a chondroitin sulfate-glutamine complex, and d) a chondroitin sulfate-arginine complex, according to one embodiment of the present invention.
FIG. 2 is a set of graphs showing the FT-IR analysis results of chondroitin sulfate, an amino acid, a simple mixture of chondroitin sulfate and an amino acid, and a chondroitin sulfate-amino acid complex, according to an embodiment of the present invention.

### [Modes of the Invention]

The present invention provides a cosmetic composition for skin improvement, including a glycosaminoglycans (GAGs)-amino acid complex as an active ingredient.

Hereinafter, the present invention will be described in detail with reference to the following examples. However, the following examples are only for exemplifying the present invention, and the content of the present invention is not limited by the following examples.

The preparation examples of a GAGs-amino acid complex (GAGs-AA complex) according to the present invention will be illustrated below.

### Preparation Example 1. Preparation of GAGs-AA complex

### Example 1. Preparation of chondroitin sulfate-arginine complex (CS-R complex)

1.75 g of arginine was introduced into 93.25 g of purified water and the resulting mixture was dissolved by stirring at 750 rpm at 50 to 60°C for 5 minutes. When all the arginine was dissolved and the solution became transparent, 5 g of sodium chondroitin sulfate was added thereto while maintaining the temperature at 45 to 50°C, and the resulting mixture was stirred at 1000 rpm for 10 minutes. When all the chondroitin sulfate was dissolved and the solution turned pale yellow, it was homogenized in a homomixer at 5,500 rpm for 5 to 15 minutes to prepare a chondroitin sulfate-arginine complex (CS-R complex).

### Example 2. Preparation of chondroitin sulfate-glutamine complex (CS-Q complex)

1.5 g of glutamine was introduced into 93.5 g of purified water and the resulting mixture was dissolved by stirring at 750 rpm at 50 to 60°C for 5 minutes. When all the glutamine was dissolved and the solution became transparent, 5 g of sodium chondroitin sulfate was added thereto while maintaining the temperature at 45 to 50°C, and the resulting mixture was stirred at 1000 rpm for 10 minutes. When all the chondroitin sulfate was dissolved and the solution turned pale yellow, it was homogenized in a homomixer at 5,500 rpm for 5 to 15 minutes to prepare a chondroitin sulfate-glutamine complex (CS-Q complex).

### Comparative Example 1. Preparation of chondroitin sulfate-arginine simple mixture (CS/R mixture)

5 g of sodium chondroitin sulfate was introduced into 40 g of purified water and the resulting mixture was dissolved by stirring at 750 rpm at room temperature for 5 minutes. In another container, 1.75 g of arginine was introduced into 53.25 g of purified water, the resulting mixture was dissolved by stirring at 750 rpm at 50 to 60°C for 5 minutes, and then the resulting solution was left to stand until it reached room temperature. Subsequently, the aqueous sodium chondroitin sulfate solution was added to the solution in which arginine had been dissolved, and the resulting mixture was stirred at 1000 rpm for 10 minutes to prepare a chondroitin sulfate-arginine simple mixture (CS/R mixture).

### Comparative Example 2. Preparation of chondroitin sulfate-glutamine simple mixture (CS/Q mixture)

5 g of sodium chondroitin sulfate was introduced into 40 g of purified water and the resulting mixture was dissolved by stirring at 750 rpm at room temperature for 5 minutes. In another container, 1.5 g of glutamine was introduced into 53.5 g of purified water, the resulting mixture was dissolved by stirring at 750 rpm at 50 to 60°C for 5 minutes, and then the resulting solution was left to stand until it reached room temperature. Subsequently, the aqueous sodium chondroitin sulfate solution was added to the solution in which glutamine had been dissolved, and the resulting mixture was stirred at 1000 rpm for 10 minutes to prepare a chondroitin sulfate-glutamine simple mixture (CS/Q mixture).

### Experimental Example 1: Analysis of physicochemical properties of GAGs-amino acid complex

### <Experimental Example 1-1> Morphological analysis of GAGs-amino acid complex

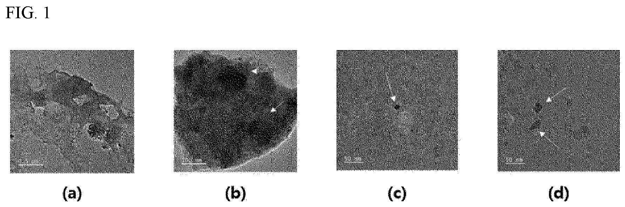

To observe whether GAGs-amino acid complexes formed specific structures, Examples 1 and 2 (chondroitin sulfate-amino acid complex (CS-AA complex)), Comparative Example 2 (simple mixture of chondroitin sulfate and glutamine (CS/Q mixture)), and chondroitin sulfate (CS) were prepared. The structure of each of these materials was analyzed using a transmission electron microscope (TEM) (JEM-F200, JEOL Ltd., Japan). The results are shown in FIG. 1 below.

As illustrated in FIG. 1, chondroitin sulfate (CS) (Comparative Example 3) was not observed to have a particulate form, but was observed to have a wide, large wall-like aggregated structure (FIG. 1A). This is because chondroitin sulfate (CS) is a polymer and the molecules are also present to be aggregated with each other. It could be confirmed that a simple mixture of chondroitin sulfate and glutamine (CS/Q mixture) (Comparative Example 2) was aggregated to have a large size of approximately 1 µm, and thus showed an aspect similar to that of Comparative Example 3 (FIG. 1B). In contrast, it could be confirmed that the chondroitin sulfate-glutamine complex (CS-Q complex) (Example 2) contained almost no aggregated material and was present in the form of very small particles (FIG. 1C). Based on these findings, as a result of preparing and analyzing a chondroitin sulfate-arginine complex (CS-R complex) (Example 1), a complex exhibiting the same aspect as in Example 2 could be confirmed (FIG. 1D). In other words, it could be confirmed that when a chondroitin sulfate-amino acid complex (CS-AA complex) was formed, the complex was present as small particles, unlike a simple mixture of chondroitin sulfate and an amino acid.

### <Experimental Example 1-2> Zeta-potential analysis of GAGs-amino acid complex

To analyze the binding characteristics in the complexes of Examples 1 and 2, particle size analysis and zeta-potential analysis were performed. Chondroitin sulfate (CS), arginine (R), and glutamine (Q), a simple mixture of chondroitin sulfate and arginine or glutamine (CS/R or CS/Q mixture), and a complex of chondroitin sulfate and arginine or glutamine (CS-R or CS-Q complex) were analyzed by dynamic light scattering using a Zetasizer Nano-ZS (Malvern Instruments, Worcester, UK) to measure the particle size (effective dynamic diameter), particle size distribution and zeta potential of the samples dispersed on a colloid. Samples for the measurements were prepared by being dispersed in distilled water, and an automatic temperature-controlled cell with a scattering angle of 90° and software provided by the manufacturer were used. After measurement was conducted in triplicate for each sample, a zeta-potential value was calculated, and the results are shown in the following Table 1.

**[Table 1]**

| Analyte | Zeta potential (mV) |
|---|---|
| Chondroitin sulfate (CS) | -18 mV |
| Arginine (R) | -4 mV |
| Comparative Example 1 | -13.6 mV (75%), 44.2 mV (24%) |
| Example 1 | No detection |
| Glutamine (Q) | 33 mV (70%), -90 mV (30%) |
| Comparative Example 2 | -21 mV (39%), 16.3 mV (16%) |
| Example 2 | No detection |

As shown in Table 1, it could be confirmed that chondroitin sulfate (CS) exhibited a zeta-potential of approximately -20 mV due to a sulfate functional group, and particles were large and aggregated. In contrast, the particle sizes of arginine (R) and glutamine (Q) were measured to be smaller than that of chondroitin sulfate (CS). In the case of zeta potential, the zeta potentials were either close to '0' or showed both (+) and (-) values because both arginine and glutamine have -NH₂ and -COOH functional groups at the same time.

In the case of the simple mixtures of Comparative Examples 1 and 2, from the measurement of two types of zeta-potential values of the (-) zeta-potential value of chondroitin sulfate (CS) and the (+) zeta-potential value of arginine (R) or glutamine (Q), it could be seen that chondroitin sulfate (CS) and an amino acid (AA) partially interact with each other, but maintain their respective properties.

In contrast, in the case of the complex, the particle size was measured to be the smallest, and the zeta potential was not measured, so it could be seen that a complex with properties different from those of chondroitin sulfate (CS) was formed.

In other words, like the transmission electron microscope image in Experimental Example 1-1, it could be confirmed even by the dynamic light scattering method that the chondroitin sulfate-amino acid complex (CS-AA complex) is present as small particles, and from the zeta-potential measurement results, it could be confirmed that this complex had different properties from either chondroitin sulfate (CS) or an amino acid.

### <Experimental Example 1-3> FT-IR analysis of GAGs-amino acid complex

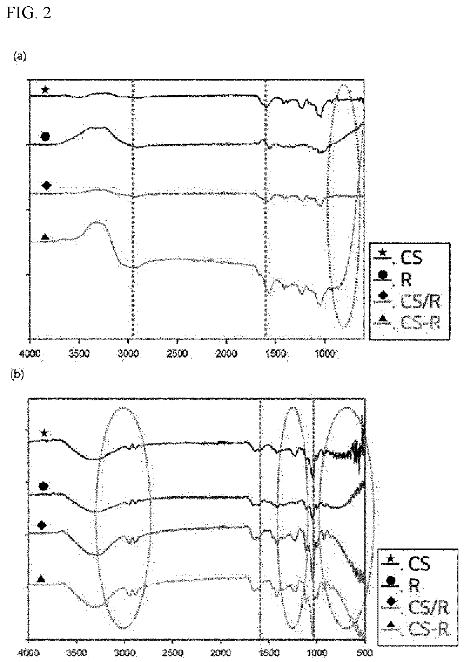

The single components, Comparative Examples 1 and 2 (simple mixtures), and Examples 1 and 2 (complexes) were analyzed by Fourier transform infrared spectroscopy (FT-IR, Spectrum Two, Perkin Elmer) to confirm whether any interactions occurred between the functional groups. The FT-IR spectrum of each sample is shown in FIG. 2.

From the observation of FIG. 2A, which compares the FT-IR spectra of chondroitin sulfate (CS), arginine (R), a simple mixture of chondroitin sulfate and arginine (CS/R mixture), and a chondroitin sulfate-arginine complex (CS-R complex), it can be confirmed that a peak shift occurs around 1600 cm⁻¹ as the complex is formed. According to the FT-IR peak table, this position corresponds to a C=O functional group adjacent to an NH amide functional group, and it is determined that a peak shift occurred while the energy of the functional group was changed by the interaction between the C=O functional group of chondroitin sulfate (CS) and the -NH₂ or =NH functional group of arginine (R). In addition, it can be confirmed that the spectrum of the complex is different from those of the other comparative groups at 1000 cm⁻¹ or less and around 3000 cm⁻¹. The region at 1000 cm⁻¹ or less is a part including - OH and -NH functional groups, and is considered to show the effect of the interaction between the -OH functional group in chondroitin sulfate (CS) and the -NH functional group in arginine (R). Furthermore, the region at around 3000 cm⁻¹ is related to the -NH functional group attached to the ring structure, so it is predicted that the energy of the -NH functional group attached to the ring structure of the complex has been changed.

From the observation of FIG. 2B, which compares the FT-IR spectra of chondroitin sulfate (CS), glutamine (Q), a simple mixture of chondroitin sulfate and glutamine (CS/Q mixture), and a chondroitin sulfate-glutamine complex (CS-Q complex), it can be confirmed that an N-H stretching (amine salt) peak at around 3000 cm⁻¹ and an S=O stretching (sulfate) peak at around 1250 cm⁻¹ experience a peak shift. This is considered to be the result of the interaction between the functional groups of chondroitin sulfate (CS) and glutamine (Q), as in FIG. 2A.

In other words, it could be confirmed by the FT-IR spectrum that an interaction between the functional groups of chondroitin sulfate (CS) and an amino acid is present.

From the summary of the results of Experimental Examples 1-1 to 1-3, it could be concluded that the chondroitin sulfate-amino acid complex (CS-AA complex) is a small particle formed by the interaction between chondroitin sulfate (CS) and an amino acid through hydrogen bonds or charge-charge interactions, and that this complex has properties different from those of each of its constituent components.

### Experimental Example 2: Evaluation of skin improvement efficacy of GAGs-amino acid complex in vitro

For the evaluation of *in vitro* skin improvement efficacy, chondroitin sulfate (CS), glutamine (Q), arginine (R), Examples 1 and 2 (CS-R complex and CS-Q complex), and Comparative Examples 1 and 2 (CS/R mixture and CS/Q mixture) were used as culture solutions, and were ultimately included at the concentrations shown in the following Table 2 in each evaluation.

**[Table 2]**

| Analyte | Concentration |
|---|---|
| Chondroitin sulfate (CS) | 1000 ppm |
| Arginine (R) | 350 ppm |
| Comparative Example 1 | CS 1000 ppm, R 350 ppm |
| Example 1 | CS 1000 ppm, R 350 ppm |
| Glutamine (Q) | 300 ppm |
| Comparative Example 2 | CS 1000 ppm, Q 300 ppm |
| Example 2 | CS 1000 ppm, Q 300 ppm |

### <Experimental Example 2-1> Confirmation of collagen synthesis effect in vitro

To compare the wrinkle amelioration effects of chondroitin sulfate (CS), arginine (R), and glutamine (Q), a simple mixture of chondroitin sulfate and an amino acid (CS/AA mixture), and a chondroitin sulfate-amino acid complex (CS-AA complex), the effect of promoting the synthesis of type I collagen was confirmed using skin fibroblasts. For the evaluation, procollagen type 1-C propeptide (P1CP), which is typically synthesized in the highest amount in fibroblasts, was quantified.

Specifically, human fibroblasts were aliquoted at 1×10⁵ cells/well in a 48-well culture plate and cultured in a Dulbecco's Modified Eagle's Medium (DMEM, Gibco) medium containing 10% fetal bovine serum (FBS) for 24 hours. Subsequently, the medium was replaced with a serum-free DMEM medium containing 2 wt% of each culture solution according to Table 2 to treat cells with an analyte for 48 hours. Next, 20 µl of the culture supernatant was taken to measure the degree of collagen increase at each concentration at 450 nm using a Procollagen Type-1-C-peptide Enzyme Immuno Assay (P1CP EIA) kit. A negative control was used as an experimental group that did not include a component of which an effect was to be confirmed, and the collagen synthesis ability value of the sample was calculated as (the value of the sample treatment group - the value of the negative control) ÷ the value of the negative control × 100. The results are shown in the following Table 3.

**[Table 3]**

| Analyte | Collagen type 1 synthesis ability |
|---|---|
| Chondroitin sulfate (CS) | 3.55 |
| Glutamine (Q) | 2.78 |
| Arginine (R) | -12.58 |
| CS/Q mixture | 5.79 |
| CS/R mixture | 14.19 |
| CS-Q complex | 19.55 |
| CS-R complex | 38.44 |
| TGF-b (positive control) | 21.15 |

As shown in the above results, collagen synthesis was remarkably increased in the complexes (Examples 1 and 2) compared to the single component or simple mixture (Comparative Examples 1 and 2). Therefore, the composition of the present invention can exhibit the effects of ameliorating skin wrinkles, improving skin elasticity, and improving dermis density through collagen synthesis.

### <Experimental Example 2-2> Confirmation of cell activity effect in vitro

To compare the effects of chondroitin sulfate (CS), arginine (R), and glutamine (Q), a simple mixture of chondroitin sulfate and an amino acid (CS/AA mixture), and a chondroitin sulfate-amino acid complex (CS-AA complex) on skin cell activity, human fibroblast culture was used to confirm whether cell activity was increased.

Specifically, human fibroblasts were aliquoted at 5×10³ cells/well and cultured in a DMEM medium containing 10% FBS for 24 hours. Subsequently, the culture medium was removed, and the cells were treated with each sample at a predetermined concentration according to Table 2 for 48 hours. Then, a reagent provided in a CCK-8 kit (DOJINDO, Cell Counting Kit-8, catalog No. CK04) was added, the resulting mixture was incubated for 4 hours, and then absorbance was measured at 450 nm using an ELISA reader. A negative control was used as an experimental group that did not include a component of which an effect was to be confirmed, and the cell activity ability value of the sample was calculated as (the value of the sample treatment group - the value of the negative control) ÷ the value of the negative control × 100. The results are shown in the following Table 4.

**[Table 4]**

| Analyte | Cell activity ability |
|---|---|
| Chondroitin sulfate (CS) | 8.74 |
| Glutamine (Q) | -11.66 |
| Arginine (R) | -12.79 |
| CS/Q mixture | 11.80 |
| CS/R mixture | 11.81 |
| CS-Q complex | 24.37 |
| CS-R complex | 18.93 |
| 5% FBS (positive control) | 26.25 |

As shown in the above results, cell activity was remarkably increased in the complexes (Examples 1 and 2) compared to the single component or simple mixture (Comparative Examples 1 and 2). Therefore, the composition of the present invention can exhibit wound healing and skin barrier recovery effects through cell activity.

### <Experimental Example 2-3> Confirmation of hyaluronic acid synthesis effect in vitro

To compare the skin moisturizing effects of chondroitin sulfate (CS), arginine (R), and glutamine (Q), a simple mixture of chondroitin sulfate and an amino acid (CS/AA mixture), and a chondroitin sulfate-amino acid complex (CS-AA complex), human dermal fibroblasts were used to confirm whether the production of hyaluronic acid was increased.

Specifically, fibroblasts were cultured in a DMEM medium containing 10% FBS until the cells occupied 80% of the culture dish. Subsequently, the cells were treated with each sample at a predetermined concentration according to Table 2 for 24 hours, and then the concentration of hyaluronic acid was measured using a Hyaluronan ELISA kit (R&D systems, catalog No. DY3614-05). A negative control was used as an experimental group that did not include a component of which an effect was to be confirmed, and the hyaluronic acid synthesis ability value of the sample was calculated as (the value of the sample treatment group - the value of the negative control) ÷ the value of the negative control × 100. The results are shown in the following Table 5.

**[Table 5]**

| Analyte | Hyaluronic acid synthesis ability |
|---|---|
| Chondroitin sulfate (CS) | 1.8 |
| Glutamine (Q) | 2.8 |
| Arginine (R) | -2.1 |
| CS/Q mixture | 7.0 |
| CS/R mixture | 19.3 |
| CS-Q complex | 14.0 |
| CS-R complex | 32.2 |
| Retinoic acid (positive control) | 16.8 |

As shown in the above results, the synthesis of hyaluronic acid was remarkably increased in the complexes (Examples 1 and 2) compared to the single components. Therefore, the composition of the present invention can exhibit a skin moisturizing effect through hyaluronic acid synthesis.

### <Experimental Example 2-4> Confirmation of suppressive effect on production of inflammatory mediators in vitro

To compare the skin soothing effects of chondroitin sulfate (CS), arginine (R), and glutamine (Q), a simple mixture of chondroitin sulfate and an amino acid (CS/AA mixture), and a chondroitin sulfate-amino acid complex (CS-AA complex), mouse macrophage cell line Raw 264.7 cells (ATCC, catalog No. CRL-2788) was used to confirm whether the production of NO was suppressed.

Specifically, Raw264.7 cells were aliquoted at 1 to 2×10⁵ cells/ml in a 24-well tissue culture plate and cultured in a DMEM medium containing 10% FBS for 24 hours. The culture was carried out in a 5% CO₂ incubator. Subsequently, the medium was replaced with a serum-free medium, and the cells were starved for 12 hours, and then treated with each sample at a predetermined concentration according to Table 2 for 30 minutes. After the sample treatment was completed, the cells were treated with 500 ng/ml lipopolysaccharides (LPS, SIGMA, catalog No. L43901) and then cultured for an additional 18 hours. Next, the culture supernatant was taken, transferred to a 96-well tissue culture plate and reacted with GREISS reagent (SIGMA, catalog No. G4410) at room temperature for 15 minutes, and then absorbance was measured at 540 nm using an ELISA reader. Thereafter, NO was quantified using the measured absorbance, and the reduction rate of NO produced in the experimental group compared to the positive control treated with 20 µg/ml of L-NMMA was calculated as (the value of the positive control - the value of the sample treatment group) ÷ the value of the negative control × 100, which was used as a NO production suppression ability. The results are shown in the following Table 6.

**[Table 6]**

| Analyte | NO production suppression ability |
|---|---|
| Chondroitin sulfate (CS) | 25.6 |
| Glutamine (Q) | 20.4 |
| Arginine (R) | 30.3 |
| CS/Q mixture | 34.2 |
| CS/R mixture | 24.3 |
| CS-Q complex | 41.5 |
| CS-R complex | 326. |

As shown in the above results, it was confirmed that the NO suppression ability was remarkably increased in the complexes (Examples 1 and 2) compared to the single component or simple mixture (Comparative Examples 1 and 2). Therefore, the composition of the present invention can exhibit anti-inflammatory, skin soothing and skin trouble ameliorating effects through NO suppression.

### <Experimental Example 2-5> Confirmation of efficacy of mRNA expression of tight junction protein in vitro

To compare the skin barrier recovery effects of chondroitin sulfate (CS), glutamine (Q), a simple mixture of chondroitin sulfate and glutamine (CS/Q mixture), and a chondroitin sulfate-glutamine complex (CS-Q complex), it was confirmed whether the expression of Zonula Occludens-1 (ZO-1), one of the proteins that make up the tight junctions at the intercellular junctions in the skin barrier, was increased.

Specifically, human keratinocyte cell line HaCaT cells were first cultured at 37°C in a 5% CO₂ environment in a DMEM medium (Gibco, Waltham, MA, USA) supplemented with 10% fetal bovine serum (FBS, Gibco, Waltham, MA, USA), 100 mg/ml penicillin, and 100 mg/ml streptomycin. Subsequently, the cultured cells were seeded onto 6-well plates at 4.0×10⁵ cells per well, and then cultured for 24 hours, the medium was replaced with a DMEM medium without FBS, and the cells were treated with each sample at a predetermined concentration according to Table 2. Moreover, the cells were again cultured for an additional 24 hours and washed twice with PBS, and then RNA was extracted using a Qiagen RNeasy Mini kit. The concentration of the extracted RNA was quantified, and 1 µg of RNA was reacted with a cDNA synthesis kit (ET21100, PhileKorea Technology) at 42°C for 30 minutes and 72°C for 10 minutes to synthesize cDNA. The synthesized cDNA was mixed with Taqman Universal Master Mix II, with UNG (4440038, Applied Biosystems) and Taqman Probe (4331182, Applied Biosystems) for reaction in a StepOnePlus Real-Time PCR System (Applied Biosystems). Through the software analysis results of the device, the relative expression level of a tight junction protein Zonula Occludens-1 (ZO-1) in terms of mRNA was calculated, and in detail, a negative control was used as an experimental group that did not include a component of which an effect was to be confirmed, and the ZO-1 expression ability value of the sample was calculated as (the value of the sample treatment group - the value of the negative control) ÷ the value of the negative control × 100. The results are shown in the following Table 7.

**[Table 7]**

| Analyte | ZO-1 expression ability |
|---|---|
| Chondroitin sulfate (CS) | 25.0 |
| Glutamine (Q) | 11.0 |
| CS/Q mixture | 10.0 |
| CS-Q complex | 56.0 |

As shown in the above results, it was confirmed that the complex (Example 2) had an excellent ability to increase the mRNA expression level of the tight junction protein ZO-1 compared to the single component or simple mixture (Comparative Example 1). Therefore, the composition of the present invention can exhibit the effect of strengthening, improving or restoring skin barriers by increasing the expression of the tight junction protein ZO-1.

### <Experimental Example 2-6> Evaluation of efficacy of GAGs-amino acid complex (CS-AA complex) in increasing beneficial bacteria of skin

To evaluate the prebiotic ability of Example 2, the degrees of enhancement in beneficial bacteria among the skin resident flora were compared before and after the CS or Example 2 treatment. *Staphylococcus epidermidis* was selected as a representative beneficial bacterium, and the growth rates of the beneficial bacterium were compared before and after treatment with the materials.

The growth rate of the beneficial bacterium was evaluated by the following method. A sterile liquid medium was prepared using a commercial tryptic soy broth (TSB, DFICO) medium, and a glycerol stock of S. *epidermidis* was inoculated into the sterile liquid TSB medium and pre-cultured at 30 to 37°C for 6 to 18 hours. Such a pre-culture was performed once to twice. Thereafter, chondroitin sulfate (CS) and Example 2 were added to a sterile liquid TSB medium at each predetermined concentration according to Table 2, and the preculture solution was inoculated to culture cells at 30 to 37°C for 6 to 18 hours.

After culture, the optical density (OD) immediately after inoculation and the optical density during the stationary phase of growth were measured, and the ratio of these values (growth rate) was compared. The growth rates of the experimental groups treated with chondroitin sulfate (CS) and Example 2 were compared to the negative control, which was an experimental group that did not include a component of which an effect was to be confirmed, to determine whether the samples had a significant effect on the growth of the bacteria, and in detail, the results were calculated as (the value of the sample treatment group - the value of the negative control) ÷ the value of the negative control × 100. After the evaluation was carried out three times, the average growth rate, the standard deviation, and the like were calculated, and then the significance was verified by a t-test. The growth rate evaluation results of the beneficial bacterium are shown in the following Table 8.

**[Table 8]**

| | | |
|---|---|---|
| *S. epidermidis* growth rate (%) | Chondroitin sulfate (CS) | CS-Q complex |
| | 6.081 | 17.521 |

As shown in the above results, it was confirmed that chondroitin sulfate (CS) itself has a certain degree of beneficial bacteria-enhancing effect, but the complex (Example 2) showed a beneficial bacteria growth rate of 17.521% compared to the non-additive control, demonstrating a remarkably excellent beneficial bacteria-enhancing effect.

### Experimental Example 3: Evaluation of skin improvement efficacy of GAGs-AA complex in vivo

### <Experimental Example 3-1> Confirmation of moisturizing improvement effect in vivo

To examine the moisturizing effect of Example 2, the moisture content of the skin was measured using a corneometer (Corneometer Cm 825 Combi 3, Courage & Khazaka, Germany). A sample for in-vivo evaluation was prepared by diluting Example 2 50 times with water. A test was performed on both arms using a corneometer. An evaluation was performed on eight people, and before performing the evaluation, the subjects' arms were washed thoroughly and left to stand for 30 minutes under constant temperature and humidity conditions (25°C, humidity 40 to 50%), and then measurement was performed. The moisture content of the arm at week 0 when nothing was applied, and the moisture content of the arm after applying the prepared samples to the same site every day for two weeks and for four weeks, were measured by the same method. The results are shown in Table 9.

**[Table 9]**

| | Moisture content of skin |
|---|---|
| Week 0 | 37.65 |
| Week 2 | 39.09 |
| Week 4 | 49.19 |

As shown in the above results, it could be confirmed that the moisture content of the skin was improved by about 30% after 4 weeks of applying Example 2 according to the present invention. Therefore, it can be seen that a skin external preparation including the complex of the present invention has a skin moisturizing effect.

### <Experimental Example 3-2> Confirmation of efficacy in brightening, improving skin pigmentation, improving skin texture, and improving pores in vivo

In addition to the moisturization of Example 2, the skin state was measured using ANTERA 3D (Miravex, Ireland) to confirm various skin improvement effects such as skin brightness, texture, tone, and pores. The measurement method was in accordance with the instrument manual. A sample for in-vivo evaluation was prepared by diluting Example 2 50 times with water. The evaluation was performed by comparing the results of week 0, when no material was applied, to the results after applying a sample prepared for evaluation results for two and four weeks to the same area every day, and comparing the measured values after two and four weeks. Three measurement points were set on the cheeks on both sides of the face, and each point was measured consecutively three times and used for analysis. The skin brightness was measured using the 'Color-L*average' value, the skin texture was measured using the 'Roughness, Rq' value, and the skin pores were measured using the pore index value, and the measurement results are shown in Table 10.

**[Table 10]**

| | Skin brightness | Skin roughness | Skin pores |
|---|---|---|---|
| Week 0 | 65.67 | 5.40 | 0.0399 |
| Week 2 | 65.96 | 5.34 | 0.0262 |
| Week 4 | 68.09 | 4.83 | 0.0144 |

As shown in the above results, it could be confirmed that after 4 weeks of applying Example 2 according to the present invention, skin brightness was improved by 3.68%, skin texture was improved by 10.5%, and skin pores were ameliorated by 62.85%. Therefore, it can be seen that a skin external preparation including the complex of the present invention has a skin improvement effect.

## Claims

1. A cosmetic composition for skin improvement, comprising a glycosaminoglycans (GAGs)-amino acid complex as an active ingredient.

2. The cosmetic composition of claim 1, wherein the GAGs are at least one selected from the group consisting of chondroitin sulfate, carrageenan, dextran sulfate, dermatan sulfate, heparan sulfate, keratan sulfate, heparin sulfate, and hyaluronic acid.

3. The cosmetic composition of claim 1, wherein the amino acid is at least one selected from the group consisting of serine, proline, threonine, cysteine, glycine, alanine, valine, leucine, isoleucine, aspartic acid, glutamic acid, glutamine, arginine, lysine, tyrosine, methionine, phenylalanine, histidine, tryptophan, and carnitine.

4. The cosmetic composition of claim 1, wherein the GAGs-amino acid complex is a polyion complex.

5. The cosmetic composition of claim 4, wherein the polyion complex is formed by means of physicochemical interaction.

6. The cosmetic composition of claim 5, wherein the physicochemical interaction is hydrophobic interaction, interaction between charges, or hydrogen bond.

7. The cosmetic composition of claim 1, wherein the cosmetic composition comprises a GAGs-amino acid complex in an amount of 0.0001 to 30 parts by weight based on 100 parts by weight of the entire composition.

8. The cosmetic composition of claim 1, wherein the GAGs-amino acid complex forms a polyion complex at a molar ratio of GAGs:amino acid = 1:0.001 to 10.

9. The cosmetic composition of claim 1, wherein the skin improvement is brightening skin, ameliorating wrinkles, improving elasticity, moisturizing, improving skin barriers, enhancing beneficial bacteria of the skin, or soothing the skin.

10. A method for preparing a cosmetic composition, the method comprising:
mixing an amino acid and a solvent to prepare a first mixture;
performing first heating and stirring on the first mixture;
adding glycosaminoglycans (GAGs) to perform second heating and stirring when the first heating and stirring is completed;
homogenizing the mixture to prepare a GAGs-amino acid complex when the second heating and stirring is completed; and
adding the GAGs-amino acid complex to prepare a cosmetic composition.

11. The method of claim 10, wherein in the preparing of the first mixture, the amino acid uses at least one selected from the group consisting of serine, proline, threonine, cysteine, glycine, alanine, valine, leucine, isoleucine, aspartic acid, glutamic acid, glutamine, arginine, lysine, tyrosine, methionine, phenylalanine, histidine, tryptophan, and carnitine.

12. The method of claim 10, wherein the performing of the first heating and stirring comprises stirring at 500 to 1500 rpm at 30 to 80°C for 3 to 30 minutes.

13. The method of claim 10, wherein, in the performing of the second heating and stirring, the GAGs use at least one selected from the group consisting of chondroitin sulfate, carrageenan, dextran sulfate, dermatan sulfate, heparan sulfate, keratan sulfate, heparin sulfate, and hyaluronic acid.

14. The method of claim 10, wherein the performing of the second heating and stirring comprises adding GAGs at a molar ratio of GAGs:amino acid=1:0.001 to 10.

15. The method of claim 10, wherein the performing of the second heating and stirring comprises stirring at 500 to 1500 rpm at 30 to 80°C for 5 to 30 minutes.

16. The method of claim 10, wherein, in the preparing of the cosmetic composition, the GAGs-amino acid complex is comprised in an amount of 0.0001 to 30 parts by weight based on 100 parts by weight of the entire composition.
